(19) Europäisches Patentamt
European Patent Office
Office européen des brevets

(11) EP 4 227 949 A1

(12) EUROPEAN PATENT APPLICATION
published in accordance with Art. 153(4) EPC

(43) Date of publication:
16.08.2023 Bulletin 2023/33

(51) International Patent Classification (IPC):
**G16C 20/10** (2019.01)

(21) Application number: 22745144.0

(52) Cooperative Patent Classification (CPC):
**G16C 20/10; G06N 3/045; G06N 3/048; G06N 5/01;**
G16C 20/70

(22) Date of filing: 21.01.2022

(86) International application number:
**PCT/CN2022/073158**

(87) International publication number:
**WO 2022/161269 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 27.01.2021 CN 202110112207

(71) Applicant: Tencent Technology (Shenzhen) Company Limited
Shenzhen, Guangdong, 518057 (CN)

(72) Inventors:
• YU, Yang
Shenzhen, Guangdong 518057 (CN)
• LU, Chan
Shenzhen, Guangdong 518057 (CN)
• ZHAO, Peilin
Shenzhen, Guangdong 518057 (CN)

(74) Representative: Eisenführ Speiser
Patentanwälte Rechtsanwälte PartGmbB
Postfach 31 02 60
80102 München (DE)

(54) **RETROSYNTHESIS PREDICTION METHOD FOR COMPOUND MOLECULE, AND RELATED APPARATUS**

(57) Disclosed in the present application are a retrosynthesis prediction method for a compound molecule, and a related apparatus. The method comprises: obtaining a target molecule, and using the target molecule as a root node in a tree structure; then performing extension processing on a first leaf node by means of a target retrosynthesis model so as to obtain a plurality of second leaf nodes; further, performing recursive processing on a predicted molecule set corresponding to the second leaf nodes, and determining an end node that satisfies a preset condition; and further, traversing path information corresponding to the end node so as to determine a retrosynthesis path of the target molecule. Therefore, the retrosynthesis prediction process of a multi-step reaction is achieved, and using step-by-step recursive extension and screening the leaf nodes can ensure the reliability of a reactant determined by means of the retrosynthesis prediction process of the multi-step reaction, and improve the accuracy of the retrosynthesis prediction of the compound molecule.

FIG. 3

## Description

This application claims priority to Chinese Patent Application No. 2021101122078, entitled "RETROSYNTHESIS PREDICTION METHOD FOR COMPOUND MOLECULE, AND RELATED APPARATUS" and filed on January 27, 2021, which is incorporated herein by reference in its entirety.

## FIELD

The present disclosure relates to the field of artificial intelligence technology, and in particular, to a technology for predicting retrosynthesis of a compound molecule.

## BACKGROUND

In recent years, the rapidly developing artificial intelligence technologies have gradually been introduced into various scientific fields and play an important role. In the chemical field, because chemical reactions are infinitely variable under different reaction conditions, researchers generally need a lot of time and effort to design a reasonable organic synthesis route when preparing compound molecules. Researchers could greatly improve the efficiency of their research and development of chemical molecules and other compounds if they were assisted in designing organic synthesis routes based on artificial intelligence technologies.

Currently, retrosynthesis algorithms based on artificial intelligence mainly includes a template-based retrosynthesis algorithm. In the template-based retrosynthesis algorithm, a template or rule for describing a chemical transformation rule may be obtained first. The template or rule may be manually labeled or may be extracted from an existing chemical reaction library. Chemical reactions predicted for the target molecule are then matched based on the obtained template or rule.

However, the template-based retrosynthesis algorithms generally require a large number of reaction templates, and may fail to make a prediction or may make an incorrect prediction for retrosynthesis processes without reaction templates, affecting the accuracy of prediction of retrosynthesis of compound molecules.

## SUMMARY

In view of this, the present disclosure provides a method for predicting retrosynthesis of a compound molecule and a related apparatus, which can effectively improve the accuracy of prediction of retrosynthesis of compound molecules.

A first aspect of the present disclosure provides a method for predicting retrosynthesis of a compound molecule, which may be executed by a computer device and specifically includes:

obtaining a target molecule and determining the target molecule as a root node in a tree structure, the root node being associated with a first leaf node in the tree structure, the first leaf node corresponding to a compound molecule, and the tree structure including a retrosynthetic path of the target molecule corresponding to the root node;

processing the compound molecule corresponding to the first leaf node by using a target retrosynthesis model to obtain multiple predicted molecule sets respectively corresponding to a plurality of second leaf nodes, the target retrosynthesis model including a graph neural network and a reactant generation network, the graph neural network being configured to predict a bond breaking position of the compound molecule corresponding to the first leaf node, and the reactant generation network being configured to determine a predicted molecule set based on the bond breaking position;

recursively processing the plurality of predicted molecule sets corresponding to the plurality of second leaf nodes and determining a terminal node that satisfies a preset condition; and

traversing paths corresponding to the terminal node to determine a retrosynthetic path of the target molecule.

A second aspect of the present disclosure provides an apparatus for predicting retrosynthesis of a compound molecule, including:

an obtaining unit, configured to obtain a target molecule and determining the target molecule as a root node in a tree structure; the root node being associated with a first leaf node in the tree structure, the first leaf node corresponding to a compound molecule, and the tree structure including a retrosynthetic path of the target molecule corresponding to the root node;

an expansion unit, configured to processing the compound molecule corresponding to the first leaf node by using a target retrosynthesis model to obtain multiple predicted molecule sets respectively corresponding to multiple second leaf nodes, the target retrosynthesis model including a graph neural network and a reactant generation network, the graph neural network being configured to predict a bond breaking position of the compound molecule corresponding to the first leaf node, and the reactant generation network being configured to determine a predicted molecule set based on the bond breaking position;

a processing unit, configured to recursively process the plurality of predicted molecule sets corresponding to the plurality of second leaf nodes and determine a terminal node that satisfies a preset condition; and

a prediction unit, configured to traverse paths corresponding to the terminal node to determine a retrosynthetic path of the target molecule.

A third aspect of the present disclosure provides a computer device, including: a memory, a processor, and a bus system, the memory being configured to store program code; and the processor being configured to execute the method for predicting retrosynthesis of a compound molecule according to the first aspect based on instructions in the program code.

According to a fourth aspect of the present disclosure, a computer-readable storage medium is provided, the computer-readable storage medium storing instructions, the instructions, when run on a computer, causing the computer to execute the method for predicting retrosynthesis of a compound molecule according to the first aspect.

A fifth aspect of the present disclosure provides a computer program product or a computer program, including computer instructions, the computer instructions being stored in a computer-readable storage medium. A processor of a computer device reads the computer instructions from the computer-readable storage medium and executes the computer instructions to cause the computer device to execute the method for predicting retrosynthesis of a compound molecule according to the first aspect.

As can be seen from the foregoing technical solutions, the embodiments of the present disclosure have the following advantages:

By obtaining a target molecule and determining the target molecule as a root node in a tree structure, the root node being associated with a first leaf node in the tree structure, and the tree structure including a retrosynthetic path of the target molecule; then, expanding the first leaf node through a target retrosynthesis model to obtain multiple second leaf nodes, the target retrosynthesis model including a graph neural network and a reactant generation network, the graph neural network being configured for predicting a bond breaking position of a compound molecule corresponding to the first leaf node, and the reactant generation network being configured for determining a predicted molecule set based on the bond breaking position; further, recursively processing the predicted molecule set corresponding to the second leaf nodes and determining a terminal node that satisfies a preset condition; and then, traversing path information corresponding to the terminal node to determine a retrosynthetic path of the target molecule; a retrosynthesis prediction process of a multi-step reaction is realized. Leaf nodes are gradually recursively expanded and screened, and path tracing is performed for the terminal node satisfying the preset condition, to ensure the reliability of reactants determined by the retrosynthesis prediction process of the multi-step reaction, thereby improving the accuracy of prediction of retrosynthesis of compound molecules.

## BRIEF DESCRIPTION OF THE DRAWINGS

FIG. 1 is a network architecture diagram of operation of a system for predicting retrosynthesis of a compound molecule.

FIG. 2 is an architecture diagram of prediction of retrosynthesis of a compound molecule according to an embodiment of the present disclosure.

FIG. 3 is a flowchart of a method for predicting retrosynthesis of a compound molecule according to an embodiment of the present disclosure.

FIG. 4 is a schematic diagram showing a working process of a search tree according to an embodiment of the present disclosure.

FIG. 5 is a schematic diagram of a process of predicting intermediate reactants according to an embodiment of the present disclosure.

FIG. 6 is a schematic diagram showing a working process of a target retrosynthesis model according to an embodiment of the present disclosure.

FIG. 7 is a schematic diagram of a retrosynthesis reaction process according to an embodiment of the present disclosure.

FIG. 8 is a schematic diagram showing a working process of a graph neural network according to an embodiment of the present disclosure.

FIG. 9 is a schematic diagram of string conversion according to an embodiment of the present disclosure.

FIG. 10 is a flowchart of another method for predicting retrosynthesis of a compound molecule according to an embodiment of the present disclosure.

FIG. 11 is a schematic diagram of a process of predicting retrosynthesis of a compound molecule according to an embodiment of the present disclosure.

FIG. 12 is a schematic diagram of another process of predicting retrosynthesis of a compound molecule according to an embodiment of the present disclosure.

FIG. 13 is a schematic diagram of another process of predicting retrosynthesis of a compound molecule according to an embodiment of the present disclosure.

FIG. 14 is a schematic diagram of another process of predicting retrosynthesis of a compound molecule according to an embodiment of the present disclosure.

FIG. 15 is a schematic structural diagram of an apparatus for predicting retrosynthesis of a compound molecule according to an embodiment of the present disclosure.

FIG. 16 is a schematic structural diagram of a terminal device according to an embodiment of the present disclosure.

FIG. 17 is a schematic structural diagram of a server according to embodiment of the present disclosure.

## DESCRIPTION OF EMBODIMENTS

First, some terms that may appear in embodiments of the present disclosure are explained.

Simplified molecular input line entry specification (SMILES) is a specification for unambiguously describing the structure of chemical molecules using short ASCII strings.

Transformer is a neural network model for sequential learning based on an attention mechanism.

Graph neural network (GNN) is a neural network used to process graph data, for example, a series of neural network algorithms that is performed on molecular maps.

Reaction center is a subgraph consisting of a vertex of a bond that breaks in the retrosynthesis and edges of its 1st level neighbors.

Monte Carlo Tree Search (MCTS) is a heuristic search algorithm for decision-making processes.

Atom-mapping is a procedure that establishes a one-to-one correspondence between atoms of reactants and atoms of products. It is commonly used in template-based retrosynthesis algorithms.

Basic molecule set is a compound library used to determine a predicted endpoint of a reaction in multi-step retrosynthesis, i.e., as long as a predicted reactant is in the basic molecule set, prediction of the reactant is stopped without being further decomposed for predicting synthetic paths. Based on this, the number of compounds included in the basic molecule set can determine the number of steps of a retrosynthetic route.

Root node is a search start node in a tree structure that is used in the retrosynthesis prediction of a compound to indicate a target synthetic compound.

Leaf node is a lower-layer node of the root node, and is used in the retrosynthesis prediction of a compound to indicate an intermediate compound that is predicted by a single-step or multi-step retrosynthesis.

Terminal node is a node in the tree structure that satisfies a search end condition, for example, a node that satisfies a search end condition in a Monte Carlo tree used for retrosynthesis prediction. The search end condition may be that the number of expansions of the terminal node reaches a preset value or that the terminal node is a molecule in the basic molecule set.

Retrosynthetic path is a path from the target synthetic compound to compounds involved in the reaction.

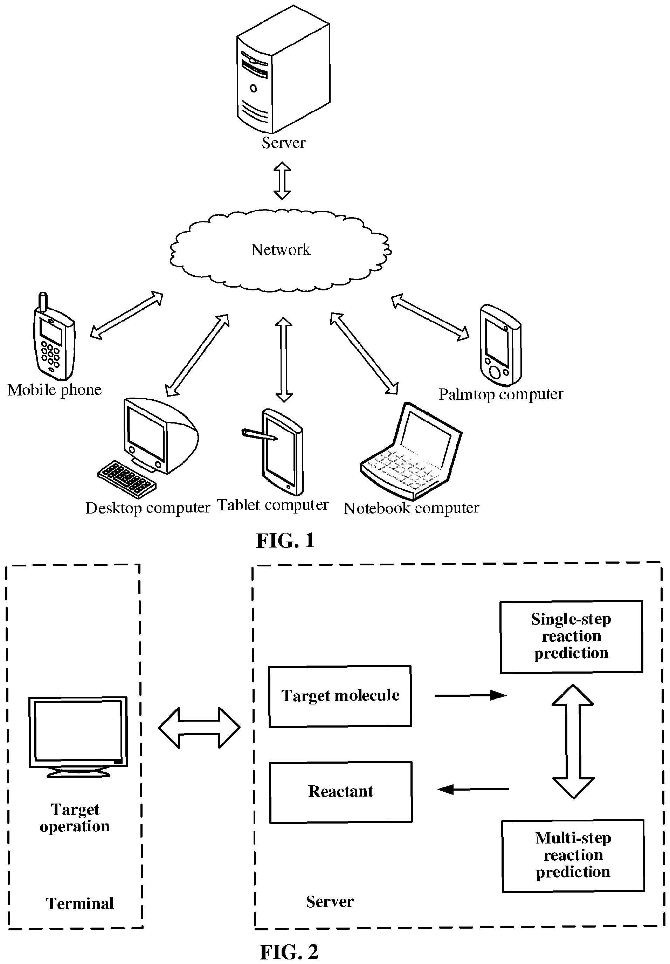

It is to be understood that the method for predicting retrosynthesis of a compound molecule according to the present disclosure may be applied to a computer device, and may be executed by a system or program running in the computer device and having a function for predicting retrosynthesis of a compound molecule, for example, a drug synthesis assistant. The system for predicting retrosynthesis of a compound molecule may be run on a network architecture shown in FIG. 1. As shown in FIG. 1, the system for predicting retrosynthesis of a compound molecule may provide a service for predicting retrosynthesis of a compound molecule for multiple information sources. For example, the target molecule may be transmitted to the server in response to an operation triggered on the terminal side, so that the server predicts a reactant and feeds back a result of the prediction to the terminal. FIG. 1 shows a variety of terminal devices. The terminal device may be a computer device. In an actual scenario, more or fewer kinds of terminal devices may participate in the process of predicting retrosynthesis of a compound molecule. The specific number and types of terminal devices are determined by the actual scenario, and are not limited here. In addition, FIG. 1 shows one server. In an actual scenario, however, multiple servers may participate in the process. The number of servers involved in the process of predicting retrosynthesis of a compound molecule is determined by the actual scenario.

In the embodiments of the present disclosure, the server may be an independent physical server, a server cluster or a distributed system including multiple physical servers, or a cloud server that provides basic cloud computing services. The terminal may be a smartphone, a tablet computer, a notebook computer, a desktop computer, a smart speaker, a smartwatch, or the like, but is not limited thereto. The terminal and the server may be directly or indirectly connected in a wired or wireless communication manner, and the terminal and the server may be connected to form a blockchain network, which is not limited in the present disclosure.

It should be understood that the system for predicting retrosynthesis of a compound molecule may be run on a personal mobile terminal, for example, as an application similar to the drug synthesis assistant; or may be run on a server, or may be run on a third-party device to predict the retrosynthesis of the compound molecule, to obtain a retrosynthesis prediction processing result of the compound molecule for an information source. The system for predicting retrosynthesis of a compound molecule may be run on the above device in the form of a program, or run on the above device as a system component, or run as a cloud service program. The specific operation mode depends on the actual scenario, and is not limited here.

Currently, the retrosynthesis prediction of a compound molecule is generally realized using template-based algorithms. However, the template-based algorithms require a large number of reaction templates, and may fail to make a prediction or may make an incorrect prediction for retrosynthesis processes without reaction templates, affecting the accuracy of prediction of retrosynthesis of compound molecules.

In order to solve the above problems, a method for predicting retrosynthesis of a compound molecule is provided according to an embodiment of the present disclosure. The method may be applied in the architecture for predicting retrosynthesis of a compound molecule shown in FIG. 2. As shown in FIG. 2, a user performs a target operation of inputting a target molecule through a terminal, so that the terminal transmits the target molecule to a server. The server predicts a reactant corresponding to the target molecule through single-step reaction prediction and multi-step reaction prediction. Molecular map structure information and SMILES string information of an original product (target molecule) are used to complement a synthon after bond breakage to obtain the reactant. The molecular map structure and the SMILES string information are fully utilized in the prediction process. In addition, in the whole process of retrosynthesis, first the bond breaking position is provided, and then synthons after the bond breakage are complemented, so that the whole process is visualized and interpretable. Subsequently, single-step results are sorted and selected using a Monte Carlo tree search method, and then multi-step reaction prediction is implemented to obtain the reactant.

It should be understood that, the method according to the present disclosure may be a program implemented as a processing logic in a hardware system or as an apparatus for predicting retrosynthesis of a compound molecule, and the above processing logic may be implemented in an integrated or external manner. As an implementation, the apparatus for predicting retrosynthesis of a compound molecule obtains a target molecule and determines the target molecule as a root node in a tree structure, the root node being associated with a first leaf node in the tree structure, and the tree structure including a retrosynthetic path of the target molecule; then, expands the first leaf node through a target retrosynthesis model to obtain multiple second leaf nodes, the target retrosynthesis model including a graph neural network and a reactant generation network, the graph neural network being configured for determining a bond breaking position of a compound molecule corresponding to the first leaf node, and the reactant generation network being configured for obtaining a predicted molecule set based on the bond breaking position; further, recursively processes the predicted molecule set corresponding to the second leaf nodes and determines a terminal node that satisfies a preset condition; and then, traverses path information corresponding to the terminal node to determine a retrosynthetic path of the target molecule. In this way, a retrosynthesis process of a multi-step reaction is predicted. In the retrosynthesis prediction process, leaf nodes are gradually recursively expanded and screened, and path tracing is performed for the terminal node satisfying the preset condition, to ensure the reliability of reactants predicted by the retrosynthesis prediction process of the multi-step reaction, thereby improving the accuracy of prediction of retrosynthesis of compound molecules.

The solutions according to the embodiments of the present disclosure involve machine learning technologies of artificial intelligence, and are described by using the following embodiments.

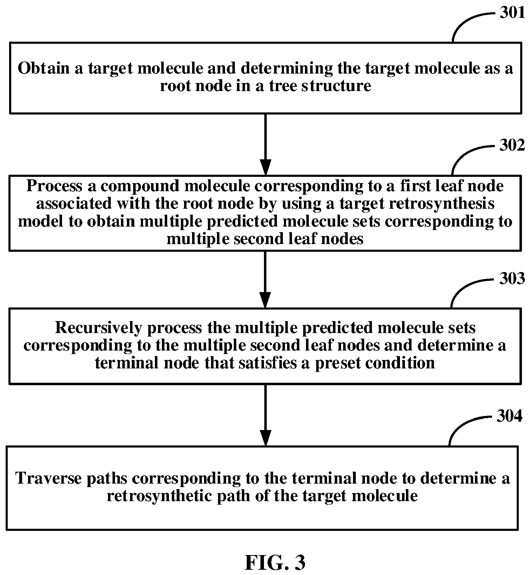

The following describes a method for predicting retrosynthesis of a compound molecule according to an embodiment of the present disclosure. Referring to FIG. 3, FIG. 3 is a flowchart of a method for predicting retrosynthesis of a compound molecule according to an embodiment of the present disclosure. The prediction method may be executed by a computer device such as a terminal or a server, or may be executed by a terminal incorporation with a server. The method according to the embodiment of the present disclosure includes at least the following steps 301 to 304.

301. Obtain a target molecule and determine the target molecule as a root node in a tree structure.

In this embodiment, the target molecule may be a drug molecule or a compound molecule with other uses. The specific molecular depends on actual scenarios.

In addition, the root node is associated with a first leaf node in the tree structure, the first leaf node corresponds to a compound molecule, and the tree structure includes a retrosynthetic path of the target molecule corresponding to the root node. The tree structure may be a Monte Carlo tree, that is, a heuristic search algorithm tree for decision-making processes. In the tree structure, the root node is a previous-layer node of the leaf node. By tracing a path from the leaf node to the root node, a synthetic path of the target molecule may be determined.

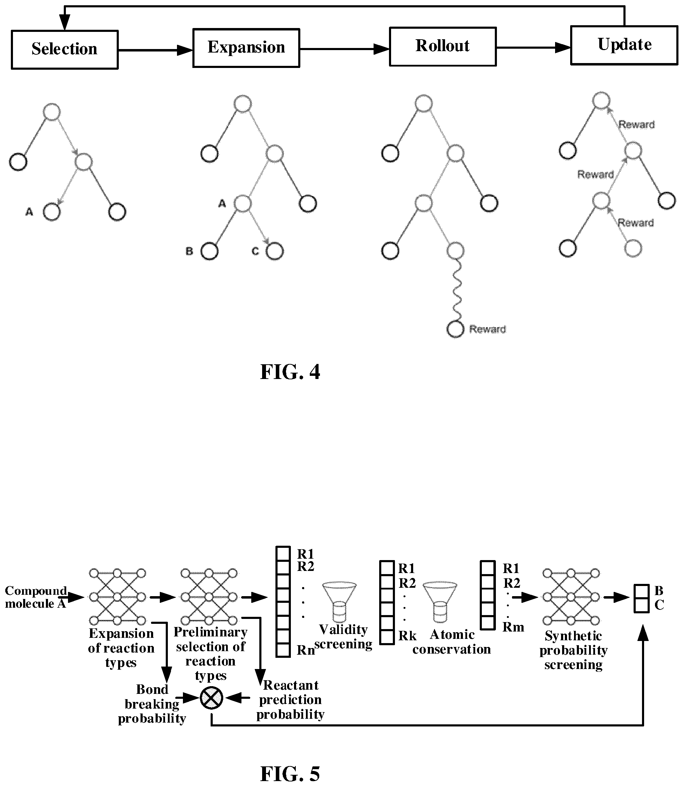

For ease of understanding, referring to FIG. 4, which is a schematic diagram showing a working process of a search tree according to an embodiment of the present disclosure, each node corresponds to a molecule set (e.g., a reactant set corresponding to a node includes multiple reactant molecules). In addition, the node corresponding to a terminal molecule (reactant) is defined as the terminal node. Starting with the root node (target molecule), the following four steps are iteratively performed on the search tree: selection, expansion, rollout, and update. After the update step is completed, the process goes back to the selection step again to repeat the above process.

The selection process includes traversing the leaf nodes of the search tree starting from the root node (target molecule). In this process, subnode A with a high score is selected. The score is generated by a scoring function which is a weighted sum of rewards fed back by all the terminal nodes expanded from the root node and a reciprocal of the number of accesses to the root node.

The expansion process includes expanding from node A with a high score to obtain node B and node C, that is, adding leaf nodes through the target retrosynthesis model. For example, 10 reaction types may be traversed first, and 3 results may be predicted for each reaction type, to obtain a total of 30 results. Subsequently, the 30 results are filtered, and some results with higher probabilities are selected using a certain rule, a retrosynthesis probability model, a forward-synthetic prediction model or other methods and are maintained. The certain rule includes: (1) the generated resultant molecule exists in nature; (2) the generated resultant molecule is an organic substance; and (3) the number of rings of the generated resultant molecule is not increased. The retrosynthetic probability model and the forward-synthetic prediction model are empirical models obtained based on synthetic experience, which reflects the feasibility of expanded compounds.

An exemplary implementation of the simulation process is as follows: 10 reaction types are traversed first, and 1 result is predicted for each reaction type, to obtain a total of 10 results. The 10 results are then filtered and the most probable reaction is selected using an inverse synthetic probability, forward-synthetic prediction probability, and the like for the subsequent expansion step. The above process is repeated until an end condition is met. The end condition may include molecules corresponding to the leaf nodes are all in the basic molecule set, or a predefined number of times is reached.

The update process includes calculating a reward of the terminal node and gradually updating the reward of an upper-level node. The reward is calculated by: obtaining a synthetic path that generates the terminal node by simulation, calculating a ratio of the number of molecules corresponding to the leaf nodes on the synthetic path in a raw material library to the number of molecules corresponding to all the leaf nodes , and updating the reward of the tree structure by using the ratio, to facilitate the execution of the subsequent selection - expansion - simulation - update cycles. Based on the tree structure, the appropriate reactant can be accurately extracted.

It can be understood that the reward in the tree structure in this embodiment is cyclically calculated, and the specific scoring function may be a scoring function used in any cycle in the cyclic process. In addition, the values described in the above steps are given by way of example only, and the specific value depends on actual scenarios.

302. Process the compound molecule corresponding to the first leaf node by using a target retrosynthesis model to obtain multiple predicted molecule sets respectively corresponding to a plurality of second leaf nodes.

In this embodiment, the target retrosynthesis model includes a graph neural network and a reactant generation network, the graph neural network being configured to predict a bond breaking position of the compound molecule corresponding to the first leaf node, and the reactant generation network being configured to determine a predicted molecule set based on the bond breaking position. That is, the process of determining intermediate reactants includes two stages: one for predicting a potential bond breaking position of the molecule and generating synthons after bond breakage; the other for complementing the synthons based on reactant information to obtain the intermediate reactants.

In an embodiment, in the process of obtaining the intermediate reactants (i.e., the process of determining the second leaf nodes), multiple candidate results may be obtained, which needs to be screened to obtain the intermediate reactants. As shown in FIG. 5, FIG. 5 is a schematic diagram of a process of predicting intermediate reactants according to an embodiment of the present disclosure. As shown in the FIG.5, after a bond breaking position of compound molecule A is determined, expansion processing is performed based on reaction types, and then a preliminary selection is performed based on reaction types to obtain a candidate sequence 1 (R1-Rn). For example, 10 reaction types with the highest scores are selected. Then, validity screening is performed to obtain a candidate sequence 2 (R1-Rk, k<n). For example, it is determined whether each molecule in candidate sequence 1 is a molecule that is effectively stable. Further, screening based on atomic conservation is performed to obtain candidate sequence 3 (R1-Rm, m<k). Synthetic probabilities in candidate sequence 3 are calculated, and screening is performed according to the synthetic probabilities to obtain possible reactant sets B and C. The synthetic probability may be obtained based on a success rate or usage rate of different types of reactions in a database.

In addition, for the determination of the intermediate reactants, bond breakage probabilities of the reactant sets obtained after the expansion based on the reaction types may be directly obtained, or reactant prediction probabilities of the reactant sets may be determined after the preliminary selection based on the reaction types, and the reactant set corresponding to the second leaf node may be determined based on the bond breakage probabilities and/or the reactant prediction probabilities.

It should be understood that, a complex molecule generally requires multiple single-step reaction predictions, and on resultant reactants or intermediate reactants obtained based on a sorting result of the single steps, a next single-step reaction prediction is performed, until reactants in a known or commercially available material library (which is defined here as a basic molecule set, and is used to determine an endpoint of the retrosynthesis analysis) is obtained. Therefore, the accuracy of single-step reaction prediction plays a crucial role in the prediction of overall multi-step retrosynthesis.

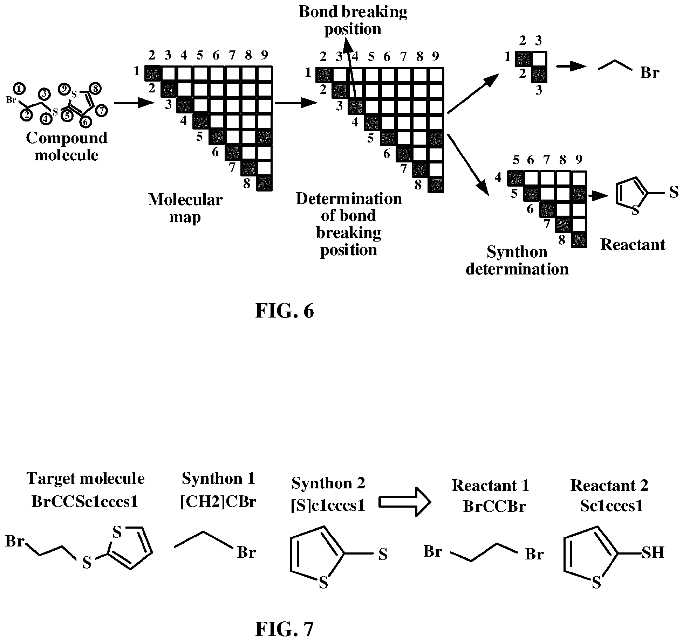

The following describes a single-step prediction process, that is, a usage process of the target retrosynthesis model. FIG. 6 is a schematic diagram showing a working process of a target retrosynthesis model according to an embodiment of the present disclosure. As shown in the FIG. 6, a single-step prediction process includes: determining a first string of the compound molecule corresponding to the first leaf node; converting the first string into a first molecular map; determining the bond breaking position of the compound molecule corresponding to the first leaf node by processing the first molecular map by using the graph neural network (determination of bond breaking position); determining at least one synthon by processing the bond breaking position by using the reactant generation network; and screening the at least one synthon based on a preset rule to determine the predicted molecule set (reactant set).

After the reactant set is obtained, a corresponding reaction process may be obtained. As shown in FIG. 7, which is a schematic diagram of a retrosynthesis reaction process according to an embodiment of the present disclosure, synthons 1 and 2 are determined from the target molecule, and reactants 1 and 2 respectively correspond to synthons 1 and 2, where reactant 1 is determined by searching the basic molecule set using synthon 1, i.e., searching for a molecule associated with synthon 1 in the basic molecule set.

It should be understood that, the bond breakage prediction is a graph-to-graph conversion issue. The process of determining the molecular map in the above steps is as follows. First, a SMILES string of a given product is converted into a corresponding molecular map Gp including Np nodes. Then, the molecular map Gp is input into the graph neural network, to predict bond breakage in the molecular map Gp.

In an embodiment, the bond breakage is predicted based on a target key feature. First, bond breaking information is obtained based on the first molecular map and a limit on the number of broken bonds by using the graph neural network; a target key feature, such as a types of an atom and a type of a bond included in the target molecule, is determined from the target molecule; and the key breaking information is extracted based on the target key feature to determine the bond breaking position.

It should be understood that, the target key feature may include an atom key feature and a bond key feature, and features of atoms (nodes) and keys (edges) used in the target key feature may be automatically extracted using the RDKit, which is an open source toolkit of collection of cheminformatics and machine-learning software written in C++ and Python, and provides a PostgreSQL-based cartridge that allows molecules to be stored in relational database and retrieved via substructure and similarity searches. The atom key feature may include at least one of an atom type, the number of bonds, a formal charge, chirality, a number of hydrogen atoms, an atomic hybridization state, aromaticity, an atomic weight, a high frequency reaction center feature, and a reaction type, and the bond key feature may include at least one of a bond type, a conjugate, a ring bond, and a molecular stereochemical feature.

The atom type is the atomic number of the atom (referring to the sequence number of an element in the periodic table). The number of bonds is the number of different chemical bonds to which the atom belongs. The formal charge is a charge of the atom assigned to the product molecule. Chirality means that a molecule cannot coincide with its mirror image, just like that a person's left hand does not coincide with the person's right hand. The number of hydrogen atoms is the number of hydrogen atoms to which the atom is attached. The atomic hybridization state includes sp, sp2, sp3, sp3d, or sp3d2. Aromaticity is used to characterize whether the atom is in an aromatic ring system. The atomic weight is the weight of the atom. The high frequency reaction center feature is used to characterize whether an atom has a high frequency reaction center feature, which depends on whether a molecular map containing the atom is a high frequency reaction center, the high frequency reaction center being a center of frequent reactions that is extracted from products of a retrosynthesis training set. The reaction type is a chemical reaction type of the retrosynthesis reaction, and may also be a feature of the atom.

The bond type is used to indicate the type of the chemical bond, such as a single bond, a double bond, a triple bond, an aromatic bond, etc. The conjugate feature is used to indicate whether the chemical bond is conjugated. The ring bond feature is used to indicate whether the chemical bond is part of a ring bond. The molecular stereochemical feature is used to indicate achirality factor, arbitrary chirality factor, double bond stereochemistry, etc.

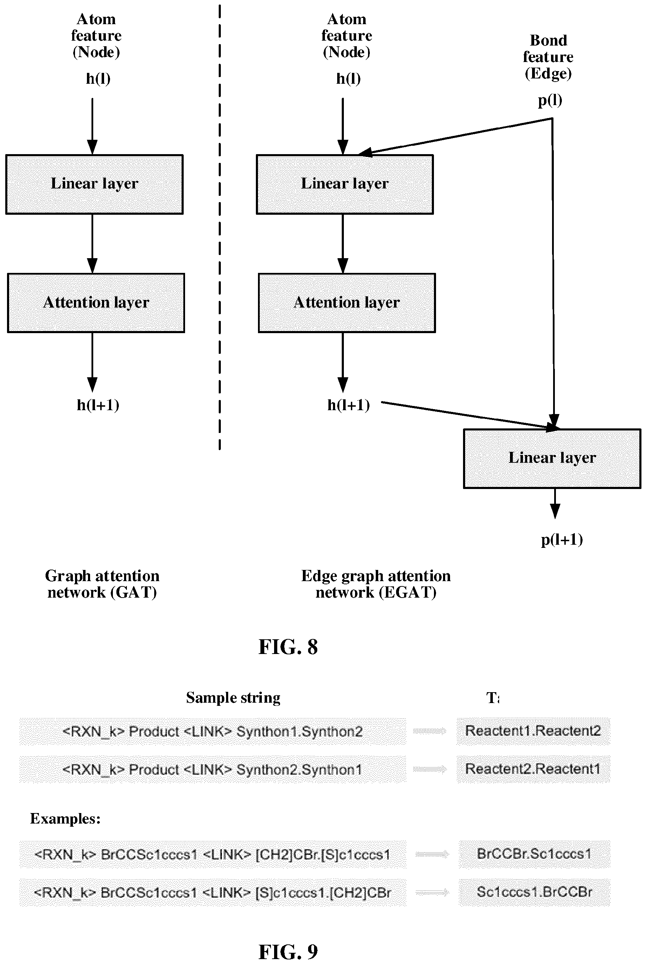

In an embodiment, since there may be multiple combinations of broken bonds for a given target molecule, an auxiliary task may be additionally performed to limit the total number of broken bonds. FIG. 8, which is a schematic diagram showing a working process of a graph neural network according to an embodiment of the present disclosure, illustrates a bond breakage prediction process (a) based on an atom feature and a bond breakage prediction process (b) based on an atom features and a bond feature. A limit is set for the number of broken bonds, the types of broken bonds or a broken bond energy value in addition to the bond breakage prediction process based on the atom feature, so that the accuracy of the determined bond breaking position can be improved.

The process of training the graph neural network in this embodiment is described below. Model training data involved in the training process may be data from subsets USPTO_50k and USPTO_480k of the dataset of 1.8 million in the US patent database from USPTO. Reaction data containing chiral molecules in the reaction is eliminated, and is processed by atom-mapping. The distribution of reaction types is shown in Table 1, mainly including 10 major chemical reaction types.

Table 1. Distribution of reaction types in USPTO_50k and USPTO_480k data

| | Reaction type | Percentage of USPTO_50k (%) | Percentage of USPTO_480k (%) |
|---|---|---|---|
| 1 | Heteroatom alkylation and arylation | 30.3 | 29.9 |
| 2 | Acylation and related pro-cesses | 23.8 | 24.9 |
| 3 | C-C bond formation | 11.3 | 13.4 |
| 4 | Heterocyclic formation | 1.8 | 0.7 |
| 5 | Protective reaction | 1.3 | 0.3 |
| 6 | Deprotection reaction | 16.5 | 14.1 |
| 7 | Reduction reaction | 9.2 | 9.4 |
| 8 | Oxidation reaction | 1.6 | 2.0 |
| 9 | Functional group intercon-version (FGI) | 3.7 | 5.0 |
| 10 | Functional group addition (FGA) | 0.5 | 0.2 |

A process of training the graph neural network includes: obtaining a first training molecule and a first training synthon corresponding to the first training molecule; determining a node feature (atom feature) and an edge feature (bond feature) of the first training molecule and the first training synthon, the node feature indicating a relationship of atoms between the first training molecule and the first training synthon, and the edge feature indicating a relationship of chemical bonds between the first training molecule and the first training synthon; determining a first loss function based on the node feature and the edge feature; determining a bond breaking probability of the chemical bond between the first training molecule and the first training synthon; determining a second loss function based on the bond breaking probability; and updating a model parameter of the graph neural network according to the first loss function and the second loss function.

The process of determining the first loss function is described as follows. a given input is provided:

$$Gp = \{Ap, Ep, Xp\}$$

where Gp is a molecular map of the first training molecule, Ap is the atom feature of the first training molecule, Ep is the bond feature of the first training molecule, and Xp is a parameter of a GAT model.

Unlike the GAT that considers only the embedding $h_i^{(l+1)}$ of nodes, an EGAT algorithm may simultaneously calculate embedding $h_i^{(l+1)}$ of nodes and $p_{i,j}^{(l+1)}$ of edges of layer *l*+1 according to embedding $h_i^{(l)}$ of nodes and $p_{i,j}^{(l)}$ of edges of layer *l* by using the following formulas.

First, a vector representation (embedding) of a node is multiplied by a weight:

$$z_i^{(l)} = W^{(l)} h_i^{(l)},$$

An activation function is then used for calculation (where a Mish function is used as an example):

$$c_{i,j}^{(l)} = Mish\left(a^{(l)^T} \left[ z_i^{(l)} \middle\| z_j^{(l)} \middle\| p_{i,j}^{(l)} \right]\right)$$ , Further, embedding of layer *l*+1 is obtained using softmax, and three vectors of the vertices i and j and an edge of layer *l*+1 are concatenated, and multiplied by a weight coefficient. A specific formula is as follows:

$$\alpha_{i,j}^{(l)} = \frac{\exp(c_{i,j}^{(l)})}{\sum_{k \in \mathcal{N}_i} \exp(c_{i,k}^{(l)})},$$

$$h_i^{(l+1)} = \sigma\left(\sum_{j \in \mathcal{N}_i} \alpha_{i,j}^{(l)} \mathbf{U}^{(l)} [z_j^{(l)} \| p_{i,j}^{(l)}]\right),$$

$$p_{i,j}^{(l+1)} = \mathbf{V}^{(l)} [h_i^{(l+1)} \| h_j^{(l+1)} \| p_{i,j}^{(l)}],$$

where, initial embedding $h_i^{(0)}$ and $p_{i,j}^{(0)}$ are inputted features of a node and an edge respectively. W, V, and U are different weight coefficients which are vectors. $z_i$ and $c_{i,j}$ are intermediate variables; $W^{(l)} \in \mathbb{R}^{F'^{(l)} \times F^{(l)}}$, $a^{(l)} \in \mathbb{R}^{2F'^{(l)} + D^{(l)}}$, $U^{(l)} \in \mathbb{R}^{F^{(l+1)} \times (F'^{(l)} + D^{(l)})}$, and $V^{(l)} \in \mathbb{R}^{D^{(l+1)} \times (2F^{(l+1)} + D^{(l)})}$ are trainable parameters (where F, F', D are dimensions of different feature vectors, respectively), $N_i$ is a neighbor node of node i, and $a_{i,j}$ is an attention parameter of node i and its neighbor j. $h_i^{(l+1)} \in \mathbb{R}^{F^{(l+1)}}$ and $p_{i,j}^{(l+1)} \in \mathbb{R}^{D^{(l+1)}}$ represents outputted representations of the node and the edge respectively, where $\mathbb{R}$ represents the real number field.

Further, bond breakage simulation losses of chemical bonds are described. After superimposing L layers of EGAT, a final representation $p_{i,j}^{(L)}$ of the edge may be obtained, representing a chemical bond between nodes i and j. $h_i^{(L)}$ represents each node. To predict a bond breakage likelihood of a bond $p_{i,j}^{(L)}$, a fully connected layer and a Sigmoid activation layer may be constructed:

$$d_{i,j} = Sigmoid(w_{fc}^T \cdot p_{i,j}^{(L)}),$$

where $d_{i,j}$, is the bond breaking position; $p_{i,j}^{(L)}$ is the final representation of the edge; and $w_{fc}^T$ is a weight coefficient.

Further, with a goal of optimizing the bond breakage prediction being to minimize broken bonds $d_{i,j}$ and the real $y_{i,j} \in \{0,1\}$, a negative logarithm of the likelihood is calculated by a binary cross entropy loss function by using the following function:

$$\mathcal{L}_{\mathrm{EGAT}} = -\frac{1}{K}\sum_{k=1}^{K}\sum_{b_{i,j}\in\mathbf{G}_k}[(1-y_{i,j})\log(1-d_{i,j}) + y_{i,j}\log(d_{i,j})],$$

where K is the amount of all data between bonds $b_{i,j}$, G is the corresponding molecular map, $y_{i,j}$ is a confidence parameter, and $d_{i,j}$ is the bond breaking position.

It should be understood that, the bond $b_{i,j}$ exists when the corresponding adjacent element $a_{i,j}$ is not zero. For ground-truth, $y_{i,j}$ = 1 means that a chemical bond between the i-th and j-th atoms breaks.

In an embodiment, a multistep approach may be used in this model training phase, but in order to improve the learning rate, preferably a training method based on cosine annealing may be used, i.e., the learning rate may be cyclically changed.

Cosine annealing reduces the learning rate by a cosine function. In the cosine function, with the increase of x, the cosine value first drops slowly, then drops at an increasing rate, and then slowly drops again. This pattern of change may be combined with the learning rate, to produce a good result using a very effective calculation method. The specific calculation formula is as follows:

$$\eta_t = \eta_{min} + \frac{1}{2}(\eta_{max} - \eta_{min})(1 + \cos(\frac{T_{cur}}{T_{max}}\pi)),$$

where $\eta_t$ is the learning rate; $\eta_{max}$ is a maximum learning rate; $\eta_{min}$ is a minimum learning rate; $T_{cur}$ is a current number of iterations; $T_{max}$ is a maximum number of iterations.

Through the above calculation process, it is possible to escape from the current local optimum and find a new local optimum. After each cycle of computation, model parameters of different local optima are saved, thereby smoothly decreasing the learning rate.

In the above embodiments, the process of using and learning the graph neural network is described. The process of using and training the reactant generation network is described below.

Synthons may be obtained by splitting the target molecule at the bond breaking position. Subgraphs of the obtained synthons may be converted into to SMILES representations through RDKit. The synthon herein is not necessarily a real reactant and may be a sub-structure of a reactant. Afterward, a transformer-based neural network for predicting reactants (reactant generation network) may be constructed.

A process of applying the reactant generation network is as follows: first, splitting the target molecule at the bond breaking position determined by using the graph neural network to obtain at least one synthon molecular map; then converting the synthon molecular map into a second string; updating the second string based on a preset reaction type to obtain a third string, where the preset reaction type may be any one of the reaction types shown in Table 1; and determining the at least one synthon by processing the third string by using the reactant generation network.

In a possible scenario, FIG. 9 is a schematic diagram of string conversion according to an embodiment of the present disclosure. Each reaction type may be represented as a string RXN. For example, a $k^{th}$ reaction type may be represented by RXN_k. Each product molecule may be expressed as a SMILES string, defined as Product. Each synthon may also be expressed as a SMILES string, defined as Synthon. The molecule corresponding to each synthon may also be expressed as a SMILES string, defined as Reactant. Source sequence data information herein is obtained by concatenating the reaction type information (if any), a canonical product SMILES string, and a corresponding synthon. A target sequence corresponds to SMILES information of a reactant of each synthon. Further, an attention mechanism may be used so that both the product and the synthon can be observed, thereby improving the interpretability of the algorithm.

In addition, during the training of the reactant generation network, the association between the training molecule, the training synthon, and the training reactant needs to be represented in a character dimension. First, a second training molecule, a second training synthon, and a training reactant may be obtained; then, a first training string is determined based on a string corresponding to the second training molecule, a string corresponding to the second training synthon, and the preset reaction type; a second training string corresponding to the training reactant is determined; further, the first training string and the second training string are associated to determine a first training sample pair; and the reactant generation network is trained based on the first training sample pair.

In a possible scenario, assuming that there are two second training synthon and that SMILES strings of the two training synthons are respectively *Synthon1* and *Synthon2,* the strings respectively corresponding to the reaction type, the product molecule, and the synthon may be combined to obtain a long string (first training string), as follows:

$$U = < RXN\_i > Product < LINK > Sython1.Synthon2$$

Then, corresponding correct target molecule strings *Reactant*1 and *Reactant2* (supposing there are only two reactants) are concatenated into a long target string (second training string):

$$V = Reactant1.Reactant2$$

Then *U* and *V* are combined to form a training sample (first training sample pair):

$$(U, V)$$

In an embodiment, in order to improve the robustness of the reactant generation network, the SMILES strings of the synthons predicted in the first stage (by bond breakage) may also be used as training data, and the prediction result may be incorrect. First, a candidate string (prediction result) predicted by the graph neural network is obtained; then the candidate string is added into the string corresponding to the second training synthon to update the first training string to a third training string; further, the third training string and the second training string are associated to determine a second training sample pair; and the reactant generation network is trained based on second training sample pair.

In a possible scenario, it is assumed that the first stage model predicts three synthons (candidate strings):

$$\widetilde{Sython1}, \widetilde{Sython2}, \widetilde{Synthon3}$$

A third training string may then be determined based on the candidate strings corresponding to the three synthons, as follows:

$$\tilde{U} = < RXN\_i > Product < LINK > \widetilde{Sython1}.\widetilde{Synthon2}.\widetilde{Synthon3}$$

Then, the third training string is combined with a correct output target V to form a sample (second training sample pair):

$$(\tilde{U}, V)$$

The reactant generation network model is then trained based on the second training sample pair.

The reactant generation network may be trained by minimizing the difference between predicted strings and strings of real reactants, with a formula being as follows:

$$\min_{\phi} L(\phi) = \frac{1}{N} \sum_{s=1}^{N} [\ell(S^s, V^s) + \ell(\tilde{S}^s, V^s)]$$

where S represent the SMILES strings of all reactants predicted by the model, s represents an index of an sth reaction sample, and $\ell$ represents any suitable loss function, e.g., a negative likelihood function.

In an embodiment, in order to reduce the difficulty of sequence model learning, the edit distance between the learning goal, i.e., SMILES expressions of reactants, and Synthons may be minimized, that is, canonical processing of SMILES of each Synthon may be performed. A target character format may be determined first, for example, using the open source chemical informatics tool RDKit; then, the first training string determined according to the string corresponding to the second training synthon and the preset reaction type is updated based on the target character format to reduce the distance between the string corresponding to the second training synthon and the first training string determined according to the preset reaction type, thereby reducing the difficulty of sequence model learning.

303. Recursively process the multiple predicted molecule sets corresponding to the multiple second leaf nodes and determine a terminal node that satisfies a preset condition.

In an embodiment, the recursive processing is a method of decomposing a problem into sub-problems of the same type by repeating an operation, thereby solving the problem. In the case of the present disclosure, the recursive processing including processing a compound in the predicted molecule set corresponding to one second leaf node by using step 302, and this process is repeated until the preset condition is met.

The recursive processing process is as follows: for a predicted molecule set among the multiple predicted molecule sets that corresponds to one of the second leaf nodes, determining a first candidate molecule that is in the predicted molecule set and that corresponds to a preset reaction type; processing the first candidate molecule by using the target retrosynthesis model to obtain multiple predicted molecule sets corresponding to multiple third leaf nodes; recursively processing the multiple predicted molecule sets corresponding to the multiple third leaf nodes to determine a second candidate molecule; and determining that a leaf node corresponding to the second candidate molecule is the terminal node in response to the second candidate molecule satisfying the preset condition, thus achieving a multi-step retrosynthesis prediction.

The preset condition may include that the molecules after the recursive processing are all molecules in the basic molecule set. That is, multiple second candidate molecules are traversed to obtain multiple pathway molecules; and it is determined that the second candidate molecule satisfies the preset condition and that the leaf node corresponding to the second candidate molecule is the terminal node, in response to the pathway molecule being a molecule in a basic molecule set. In this way, it is ensured that the predicted compound molecules on the synthetic path are all simple basic molecules, thereby ensuring the feasibility of this solution in actual scenarios.

In addition, the selection of the basic molecule set needs to consider the rationality of the basic molecule set for the prediction results of multi-step retrosynthesis. The rationality herein means that a chemist considers the price and commercial accessibility of the intermediates in each step of the synthesis route during practical synthesis and decides whether to continue to carry out retrosynthesis according the these factors. Therefore, the selection of the basic molecule set needs to ensure as much as possible that the molecules are all commercially available chemical intermediates, rather than drug molecules used for virtual screening of drugs. The difference lies in that chemical intermediates generally have large packaging specifications (in grams), while the drug molecules used for screening generally have small packaging specifications in milligrams, and are relatively expensive. Based on this principle, building block libraries of some compound reagent companies having public reagent catalogs and other companies having other molecule libraries may be selected. Molecules in building block libraries are relatively small and simple in structure, and are cost-effective in actual usage scenarios.

It should be understood that, the selection of the basic molecule set mainly considers the commercial availability of compounds and whether the compounds are known, so commonly seen compound feedstock suppliers such as eMolecules (Plus and SC libraries, greater than 20 million) and commonly seen compound library companies providing building blocks such as Enamine (97,000) and ChemDiv (70,000) may be selected. After merging and de-duplicating the libraries, a basic molecule set including about 23 million molecules is finally determined.

In an embodiment, the preset condition may be the number of times of recursive processing, i.e., a number of expansions for the second candidate molecules. First, the number of expansions corresponding to the second candidate molecule is determined in the tree structure; and it is determined that the second candidate molecule satisfies the preset condition and that the leaf node corresponding to the second candidate molecule is the terminal node, in response to the number of expansions reaching a preset value. For example, assuming that a preset number of expansions of the second candidate molecule is 2, the second candidate molecule is processed to generate a third candidate molecule, and then the third candidate molecule is further processed to generate a fourth candidate molecule, and the fourth candidate module may be determined to be the terminal node.

304. Traverse paths corresponding to the terminal node to determine a retrosynthetic path of the target molecule.

In this embodiment, the path information corresponding to the terminal node is traversed, that is, a reverse search is performed in a direction from the terminal node to the root node, the compounds involved in the path are associated, and the retrosynthesis path of the target molecule is determined.

In a possible scenario, the prediction method of multi-step retrosynthesis reaches a prediction accuracy of 64% for the top-10 on ChEMBL's 100 public molecule test sets, that is, can predict 64 out of 100 molecules; and reaches a prediction accuracy of 43% for the top-1. This result is in the leading position compared with the performance of several other public platforms.

As can be seen from the above embodiments, by obtaining a target molecule and determining the target molecule as a root node in a tree structure, the root node being associated with a first leaf node in the tree structure, and the tree structure including a retrosynthetic path of the target molecule; then, expanding the first leaf node through a target retrosynthesis model to obtain multiple second leaf nodes, the target retrosynthesis model including a graph neural network and a reactant generation network, the graph neural network being configured for predicting a bond breaking position of a compound molecule corresponding to the first leaf node, and the reactant generation network being configured for determining a predicted molecule set based on the bond breaking position; further, recursively processing the predicted molecule set corresponding to the second leaf nodes and determining a terminal node that satisfies a preset condition; and then, traversing path information corresponding to the terminal node to determine a retrosynthetic path of the target molecule; a retrosynthesis prediction process of a multi-step reaction is realized. Leaf nodes are gradually recursively expanded and screened, and path tracing is performed for the terminal node satisfying the preset condition, to ensure the reliability of reactants determined by the retrosynthesis prediction process of the multi-step reaction, thereby improving the accuracy of prediction of retrosynthesis of compound molecules.

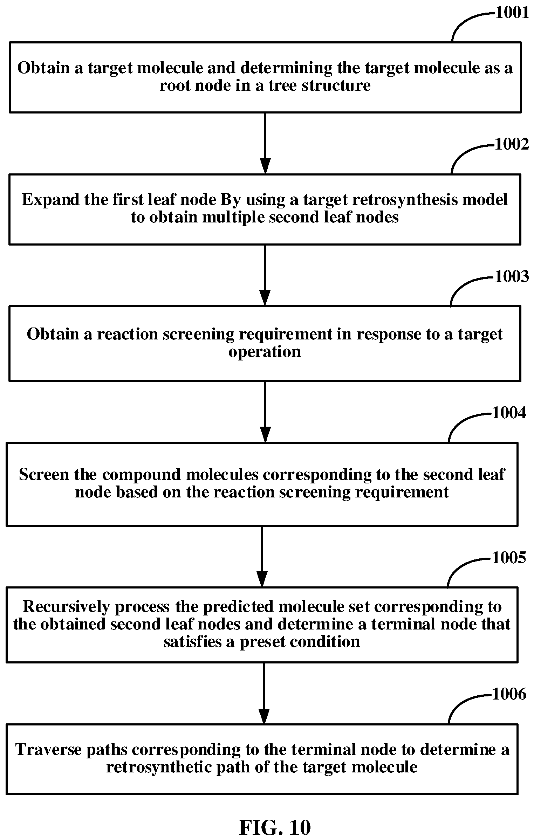

In the above embodiments, the process of predicting retrosynthesis of multi-step reactions is described. In the expansion process, different rules (for example, requiring the generated resulting molecule to be a molecule existing in nature, requiring the generated resulting molecule to be an organic substance, requiring the number of rings of the generated resulting molecule to be not increased, etc.) and models (for example, retrosynthesis probability model, forward-synthesis prediction model, etc.) are used for node screening. The rules and models may be automatically called or set by related personnel according to needs. The scenario is described below. Referring to FIG. 10, FIG. 10 is a flowchart of another method for predicting retrosynthesis of a compound molecule according to an embodiment of the present disclosure. The method according to this embodiment of the present disclosure includes at least the following steps 1001 to 1006.

1001. Obtain a target molecule and determining the target molecule as a root node in a tree structure.

1002. Expand the first leaf node, that is, process a compound molecule corresponding to the first leaf node by using a target retrosynthesis model to obtain multiple second leaf nodes.

In this embodiment, the implementation process of steps 1001-1002 is similar to the implementation process of steps 301-302 in the embodiment shown in FIG. 3. For the description of related features, reference may be made to the description of FIG. 3, and the details will not be repeated here.

1003. Obtain a reaction screening requirement in response to a target operation.

In this embodiment, the reaction screening requirement is used for subsequent screening of nodes participating in the simulation, for example, to screen out compounds having a risk of functional group interference, to select compounds having a protecting group, to screen out compounds according to the distribution characteristics of C-H bonds, or to select compounds involved in name reactions, and so on. The specific reaction screening requirement depends on actual scenarios.

It should be understood that, the target operation may be an input operation triggered by relevant personnel in the background, or may be an operation of setting a screening rule between nodes of a tree structure. The specific operation method depends on actual scenarios.

In an embodiment, the reaction screening requirement may also be applied to the screening in the expansion process. For example, in the expansion process, 10 reaction types are traversed first, and 3 results are predicted for each reaction type, to obtain a total of 30 at most. Then the 30 results are screened using the reaction screening requirement, and several results with high probabilities are selected.

1004. Screen the compound molecules corresponding to the second leaf node based on the reaction screening requirement.

In this embodiment, the reaction screening requirement is described with reference to specific reactions.

(1) Use of competitive reactions.

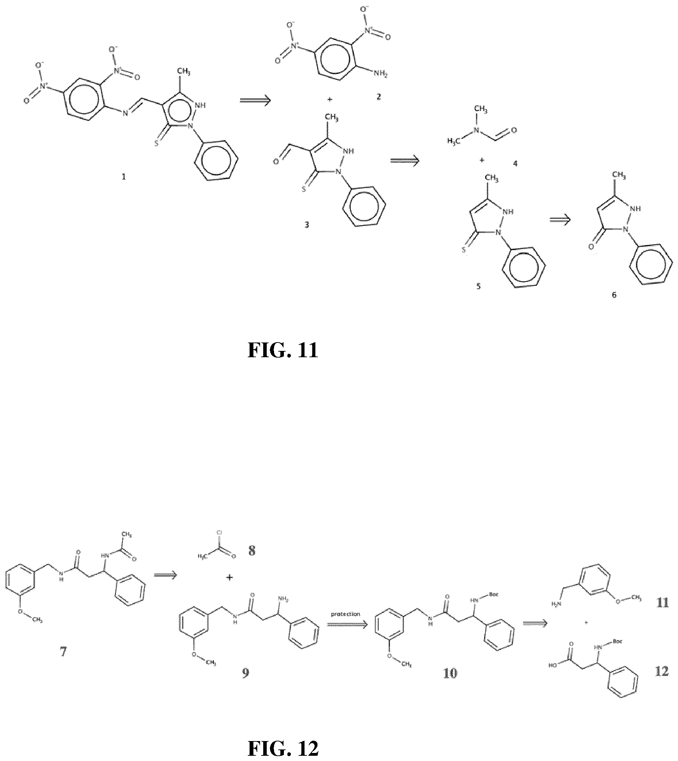

In this embodiment, the reaction route is reasonably designed by using competitive reactions to avoid functional group interference. FIG. 11 is a schematic diagram of a process of predicting retrosynthesis of a compound molecule according to an embodiment of the present disclosure. The key step in the synthetic route in the figure is to convert C=O to a C=S double bond. If a thio reaction takes place in a route predicted for compound 3, there will be a number of competitive reactions for C=O double bond, namely, aldehyde-carbonyl and amide-carbonyl competitive reactions. Therefore, nodes including the corresponding reactants need to be removed. In this way, the given route can avoid predictions including competitive reactions, i.e., the thio reaction takes place at the time when compound 5 is selected, followed by the introduction of aldehyde group to obtain compound 3, thereby improving the accuracy of retrosynthesis prediction.

(2) Use of protecting groups.

The rational use of protecting groups has been a challenge for computer-assisted retrosynthesis prediction. This is because the computer is required to analyze a group in the reaction substrate that may undergo competitive reactions, then rationally use a protecting group to protect the group that is not expected to participate in the reaction, and remove the protecting group.

In a possible scenario, FIG. 12 is a schematic diagram of a process of predicting retrosynthesis of a compound molecule according to an embodiment of the present disclosure. For target synthetic compound 9, a free NH2 group undergoes competitive reactions. Therefore, compound 10 obtained based on NHBoc is protected first, then compound 10 can be easily obtained from compounds 11 and 12; otherwise free NH2 in compound 12 will undergo an intramolecular reaction, affecting the reaction result.

In another possible scenario, hydroxyl groups often need to be protected during oxidation or in certain reactions under alkaline conditions. Specific modes of protection may include: (1) ether reactions may be used to prevent hydroxyl groups from being influenced by bases, for example: ROH + ROH→H2O + R-O-R; (2) esterification reactions may be used to prevent oxidation of hydroxyl groups.

In addition, there is sometimes a need to protect carboxyl groups under high temperature or alkaline conditions. The most commonly used method to protect carboxyl groups is esterification reactions. Alternatively, an unsaturated carbon-carbon bond is protected. A carbon-carbon double bond is easily oxidized, and is usually protected by an addition reaction to make it saturated. Alternatively, carbonyl groups, especially aldehyde groups, often need to be protected during oxidation reactions or in the presence of a base. The carbonyl group is generally protected as an acetal or ketal. The acetal or ketal may hydrolyze into the original aldehyde or ketone. The specific protecting group determined depends on actual scenarios, and is not limited herein.

(3) Application of regioselectivity.

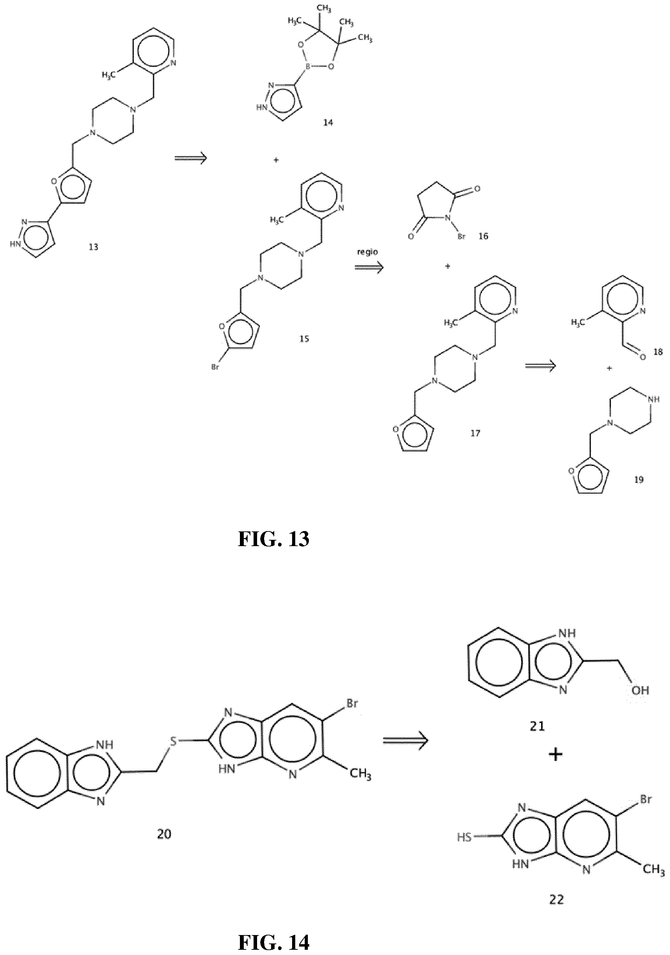

In this embodiment, the regioselectivity issue of the C-H substitution reaction on the aromatic ring is solved. FIG. 13 is a schematic diagram of a process of predicting retrosynthesis of a compound molecule according to an embodiment of the present disclosure. In predicting molecule 15, a bromo reaction of NBS (16) and compound 17 is selected. C-H (pyridine and furan rings) of multiple aromatic rings in compound 17 may be brominated by compound 16. Therefore, the rational utilization of the characteristics such as the distribution of partial charge of each C-H is the key to correctly predicting regioselectivity. Therefore, in the process of setting the reaction screening requirement, a specific C-H distribution rule may be specified, so that the corresponding reactants can be selected.

(4) Use of classic name reactions.

In this embodiment, since organic name reactions are mostly reliable chemical reactions determined by the research and use of many organic chemists, the generalizability of the substrate will also be higher, and the reaction route is relatively easy to realize. Therefore, in the retrosynthesis analysis, the use of classical name reactions is a relatively reliable route, relatively easy to be favored by users, and can predict reasonable and robust chemical reactions. FIG. 14 is a schematic diagram of a process of predicting retrosynthesis of a compound molecule according to an embodiment of the present disclosure. A Mitsunobu reaction is shown in the figure, i.e., compounds 21 and 22 are preferentially selected during the reactant screening process.

The use of classical name reactions includes, but is not limited to, the following examples:

Beckmann rearrangement: reaction of ketamine to amide (caprolactam) under acidic conditions;

Cannizzaro disproportionation: reaction of aldehyde without $\alpha$-h to give an alcohol and a carboxylic acid under a strong base (benzaldehyde);

Claisen condensation: reaction in which an ester forms a carbon anion under a strong base to undergo nucleophilic addition-elimination;

Clemmensen reduction: reduction of ketones to alkanes using zinc amalgam and concentrated hydrochloric acid (carbonyl to methylene);

Cope reaction: elimination reaction that occurs after a tertiary amine is treated with hydrogen peroxide followed by heating (Hoffmann rule);

Corey-house reaction: coupling of halogenated hydrocarbons and dialkyl copper lithium reagents (important reactions to link carbon chains);

Cram's rule: preferential nucleophilic attack takes place from the least hindered side of the carbonyl carbon;

Dickerman condensation: reaction similar to ester condensation, forming a ring;

Deers-Adel reaction: generally a reaction of a derivative of 1,3-butadiene and a derivative of ethylene (synergistic reaction);

Fehling's solution: newly prepared copper hydroxide which oxidizes an aldehyde to an acid;

Friedel-crafts: reaction for introducing an alkyl or acyl group in benzene nucleus.

It should be understood that, in an actual scenario, one or more of the above reaction screening requirements may be used. The specific number and sequencing of reaction screening requirements depend on actual scenarios.

1005. Recursively process the predicted molecule set corresponding to the obtained second leaf nodes and determine a terminal node that satisfies a preset condition.

In recursive processing, for the object simulated each time, 10 reaction types may be traversed, and 1 results may be predicted for each reaction type, to obtain a total of 10 results. The 10 results are then filtered and the most probable reaction is selected using the reaction screening requirement, an inverse synthetic probability, forward-synthetic prediction probability, and the like for the subsequent expansion step. Then the above process is repeated until an end condition is met. The end condition may be that compound molecules corresponding all the leaf nodes are in the basic molecule set, or a preset number of recursions is reached.

1006. Traverse paths corresponding to the terminal node to determine a retrosynthetic path of the target molecule.

In this embodiment, the implementation process of steps 1005-1006 is similar to the implementation process of steps 303-304 in the embodiment shown in FIG. 3. For the description of related features, reference may be made to the description of FIG. 3, and the details will not be repeated here.

It should be understood that, the setting of the above reaction screening requirement may also be applied to a single-step retrosynthesis prediction scenario. That is, the present disclosure also discloses a single-step retrosynthesis prediction method. Reference may be made to step 302 in the embodiment shown in FIG. 3 for details, which will not be repeated here.

For the single-step retrosynthesis prediction process, a prediction accuracy of 62.4% is reached even for data without reaction type labels, making the method more generalized and practical, as such reaction type labels may not be available in actual usage scenarios. It has been proved to certain extent that large datasets can improve the accuracy of single-step predictions. Specifically, a predictable accuracy of 58% is reached for a dataset of a size of 50,000, and when the size of dataset is increased to 480,000, the accuracy is increased to 62.4%.

In this embodiment, the prediction accuracy of multi-step retrosynthesis is in the leading position compared with the performance of several other existing public platforms. The prediction method of multi-step retrosynthesis reaches a prediction accuracy of 64% for the top-10 on ChEMBL's 100 public molecule test sets, that is, can predict 64 out of 100 molecules; and reaches a prediction accuracy of 43% for the top-1, with good implementability.

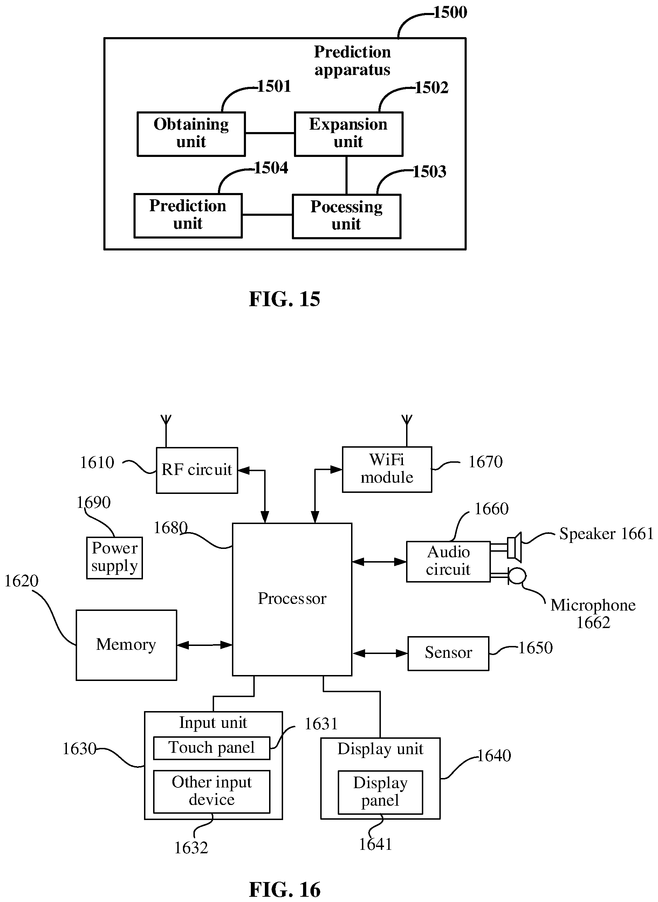

The embodiments of the present disclosure further provide a related apparatus for implementing the above solution. Referring to FIG. 15, FIG. 15 is a schematic structural diagram of an apparatus for predicting retrosynthesis of a compound molecule according to an embodiment of the present disclosure. The prediction apparatus 1500 includes:

an obtaining unit 1501, configured to obtain a target molecule and determining the target molecule as a root node in a tree structure; the root node being associated with a first leaf node in the tree structure, the first leaf node corresponding to a compound molecule, and the tree structure including a retrosynthetic path of the target molecule corresponding to the root node;

an expansion unit 1502, configured to processing the compound molecule corresponding to the first leaf node by using a target retrosynthesis model to obtain multiple predicted molecule sets respectively corresponding to multiple second leaf nodes, the target retrosynthesis model including a graph neural network and a reactant generation network, the graph neural network being configured to predict a bond breaking position of the compound molecule corresponding to the first leaf node, and the reactant generation network being configured to determine a predicted molecule set based on the bond breaking position;

a processing unit 1503, configured to recursively process the multiple predicted molecule sets corresponding to the multiple second leaf nodes and determine a terminal node that satisfies a preset condition; and

a prediction unit 1504, configured to traverse paths corresponding to the terminal node to determine a retrosynthetic path of the target molecule.

In an embodiment, in some possible implementations of the present disclosure, the expansion unit 1502 is further configured to execute operations of:

determining a first string for the compound molecule corresponding to the first leaf node;

converting the first string into a first molecular map;

determining the bond breaking position of the compound molecule corresponding to the first leaf node by processing the first molecular map by using the graph neural network;

determining at least one synthon according to the bond breaking position by using the reactant generation network; and

screening the at least one synthon based on a preset rule to determine the predicted molecule set.

In an embodiment, in some possible implementations of the present disclosure, the expansion unit 1502 is further configured to execute operations of:

determining bond breaking information by processing the first molecular map and under a limitation on a number of broken bonds by using the graph neural network; determining a target key feature of the target molecule, the target key feature including an atom key feature and a bond key feature, the atom key feature including at least one of an atom type, a number of bonds, a formal charge, chirality, a number of hydrogen atoms, an atomic hybridization state, aromaticity, an atomic weight, a high frequency reaction center feature, and a reaction type, and the bond key feature including at least one of a bond type, a conjugate, a ring bond, and a molecular stereochemical feature; and

extracting the key breaking information based on the target key feature to determine the bond breaking position.

In an embodiment, in some possible implementations of the present disclosure, the expansion unit 1502 is further configured to execute operations of:

obtaining a first training molecule and a first training synthon corresponding to the first training molecule;

determining a node feature and an edge feature of the first training molecule and the first training synthon, the node feature indicating a relationship of atoms between the first training molecule and the first training synthon, and the edge feature indicating a relationship of chemical bonds between the first training molecule and the first training synthon;

determining a first loss function based on the node feature and the edge feature;

determining a bond breaking probability of the chemical bond between the first training molecule and the first training synthon;

determining a second loss function based on the bond breaking probability; and

updating a model parameter of the graph neural network according to the first loss function and the second loss function.

In an embodiment, in some possible implementations of the present disclosure, the expansion unit 1502 is further configured to execute operations of:

splitting the target molecule at the bond breaking position to obtain at least one synthon molecular map;

converting the synthon molecular map into a second string;

updating the second string based on a preset reaction type to obtain a third string; and

determining the at least one synthon by processing the third string by using the reactant generation network.

In an embodiment, in some possible implementations of the present disclosure, the expansion unit 1502 is further configured to execute operations of:

obtaining a second training molecule, a second training synthon, and a training reactant;

determining a first training string based on a string corresponding to the second training molecule, a string corresponding to the second training synthon, and the preset reaction type;

determining a second training string corresponding to the training reactant;

associating the first training string and the second training string to determine a first training sample pair; and

training the reactant generation network based on the first training sample pair.

In an embodiment, in some possible implementations of the present disclosure, the expansion unit 1502 is further configured to execute operations of:

obtaining a candidate string predicted by the graph neural network;

adding the candidate string into the string corresponding to the second training synthon to update the first training string to a third training string;

associating the third training string and the second training string to determine a second training sample pair; and

training the reactant generation network based on the second training sample pair.

In an embodiment, in some possible implementations of the present disclosure, the expansion unit 1502 is further configured to execute operations of:

determining a target character format; and
updating the first training string based on the target character format.

In an embodiment, in some possible implementations of the present disclosure, the processing unit 1503 is further configured to execute operations of, for a predicted molecule set among the plurality of predicted molecule sets that corresponds to one of the plurality of second leaf nodes,:

determining a first candidate molecule that corresponds to a preset reaction type and that is in the predicted molecule set;

processing the first candidate molecule by using the target retrosynthesis model to obtain multiple predicted molecule sets corresponding to multiple third leaf nodes;

recursively processing the multiple predicted molecule sets corresponding to the multiple third leaf nodes to determine a second candidate molecule; and

determining that a leaf node corresponding to the second candidate molecule is the terminal node in response to the second candidate molecule satisfying the preset condition.

In an embodiment, in some possible implementations of the present disclosure, the processing unit 1503 is further configured to execute operations of:

traversing based on the second candidate molecule to obtain multiple pathway molecules; and

determining that the second candidate molecule satisfies the preset condition and determining that the leaf node corresponding to the second candidate molecule is the terminal node, in response to the pathway molecule being a molecule in a basic molecule set.

In an embodiment, in some possible implementations of the present disclosure, the processing unit 1503 is further configured to execute operations of:

determining a number of times of recursive processing corresponding to the second candidate molecule in the tree structure; and
determining that the second candidate molecule satisfies the preset condition and determining that the leaf node corresponding to the second candidate molecule is the terminal node, in response to the number of times of recursive processing reaching a preset value.

Through the above apparatus, a retrosynthesis prediction process of a multi-step reaction is realized. Leaf nodes are gradually recursively expanded and screened, and path tracing is performed for the terminal node satisfying the preset condition, to ensure the reliability of reactants determined by the retrosynthesis prediction process of the multi-step reaction, thereby improving the accuracy of prediction of retrosynthesis of compound molecules.

The embodiments of the present disclosure also provide a terminal device. FIG. 16 is a schematic structural diagram of another terminal device according to an embodiment of the present disclosure. For ease of description, only a part related to this embodiment of the present disclosure is shown. For a specific technical detail not disclosed, refer to the method part in the embodiments of the present disclosure. The terminal device may be any terminal device including a mobile phone, a tablet computer, a personal digital assistant (PDA), a point of sales (POS), and an on-board computer, and the terminal device being a computer is used as an example.

FIG. 16 is a block diagram of a structure of a part of a computer related to a terminal according to an embodiment of the present disclosure. Referring to FIG. 16, the computer includes: components such as a radio frequency (RF) circuit 1610, a memory 1620, an input unit 1630 (including a touch panel 1631 and another input device 1632), a display unit 1640 (including a display panel 1641), a sensor 1650, an audio circuit 1660 (connected to a speaker 1661 and a microphone 1662), a wireless fidelity (WiFi) module 1670, a processor 1680, and a power supply 1690. A person skilled in the art may understand that, the structure of the computer shown in FIG. 16 does not constitute a limitation to the computer device. The computer may include components that are more or fewer than those shown in the figure, or some components may be combined, or a different component deployment may be used.

The mobile phone further includes the power supply 1690 (such as a battery) for supplying power to the components. In an embodiment, the power supply may be logically connected to the processor 1680 by using a power management system, thereby implementing functions such as charging, discharging and power consumption management by using the power management system.

Although not shown in the figure, the mobile phone may further include a camera, a Bluetooth module, and the like. Details are not described herein again.

In an embodiment of the present disclosure, the processor 1680 included in the terminal is configured to execute the functions of various steps of the method for predicting retrosynthesis of a compound molecule as described above.

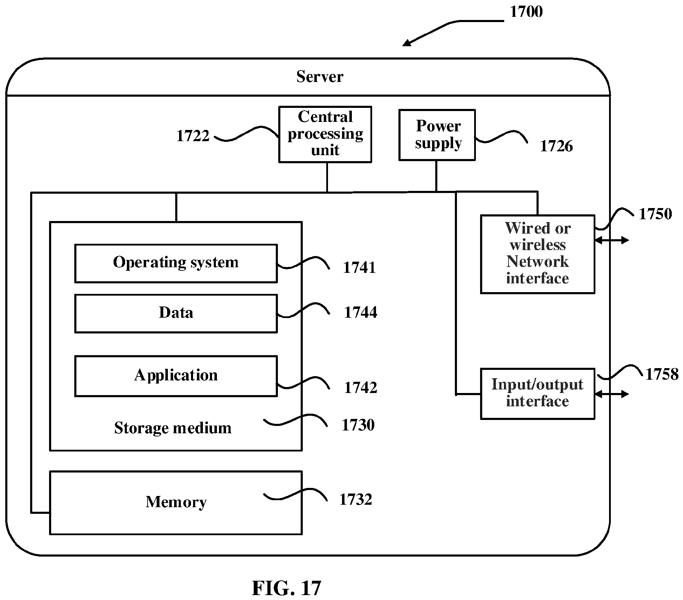

The embodiments of the present disclosure further provides a server. FIG. 17 is a schematic structural diagram of a server according to an embodiment of the present disclosure. The server 1700 may vary greatly due to different configurations or performance, and may include one or more central processing units (CPU) 1722 (for example, one or more processors) and a memory 1732, and one or more storage medium 1730 (for example, one or more mass storage devices) that store application programs 1742 or data 1744. The memory 1732 and the storage medium 1730 may be transient or persistent storages. The program stored in the storage medium 1730 may include one or more modules (not marked in the figure), and each module may include a series of instruction operations to the server. Still further, the CPU 1722 may be configured to communicate with the storage medium 1730, and perform, on the server 1700, the series of instruction operations in the storage medium 1730.

The server 1700 may further include one or more power supplies 1726, one or more wired or wireless network interfaces 1750, one or more input/output interfaces 1758, and/or one or more OSs 1741, for example, Windows Server™, Mac OS X™, Unix™, Linux™, or FreeBSD™.

The central processing unit 1722 in the server 1700 is further configured to execute the functions of various steps of the method for predicting retrosynthesis of a compound molecule as described above.

The embodiments of the present disclosure further provide a computer-readable storage medium, storing instructions for predicting retrosynthesis of a compound molecule, the instructions, when run on a computer, causing the computer to execute the steps executed by the apparatus for predicting retrosynthesis of a compound molecule in the method described in the embodiments of FIG. 3 to FIG. 14.

The embodiments of the present disclosure further provide a computer program product including instructions for predicting retrosynthesis of a compound molecule, the instructions, when run on a computer, causing the computer to execute the steps executed by the apparatus for predicting retrosynthesis of a compound molecule in the method described in the embodiments of FIG. 3 to FIG. 14.

The embodiments of the present disclosure further provide a system for predicting retrosynthesis of a compound molecule. The system for predicting retrosynthesis of a compound molecule may include the apparatus for predicting retrosynthesis of a compound molecule in the embodiment described in FIG. 15, or the terminal device in the embodiment described in FIG. 16, or the server described in FIG. 17.

Persons skilled in the art may clearly understand that, for the purpose of convenient and brief description, for a detailed working process of the system, apparatus, and unit described above, refer to a corresponding process in the method embodiments, and details are not described herein again.

The foregoing embodiments are merely intended for describing the technical solutions of the present disclosure, but not for limiting the present disclosure. It is to be understood by a person of ordinary skill in the art that although the present disclosure has been described in detail with reference to the foregoing embodiments, modifications can be made to the technical solutions described in the foregoing embodiments, or equivalent replacements can be made to some technical features in the technical solutions, as long as such modifications or replacements do not cause the essence of corresponding technical solutions to depart from the spirit and scope of the technical solutions of the embodiments of the present disclosure.

**Claims**

1. A method for predicting retrosynthesis of a compound molecule, executed by a computer device, the method comprising:

   obtaining a target molecule and determining the target molecule as a root node in a tree structure, the root node being associated with a first leaf node in the tree structure, the first leaf node corresponding to a compound molecule, and the tree structure comprising a retrosynthetic path of the target molecule corresponding to the root node;
   processing the compound molecule corresponding to the first leaf node by using a target retrosynthesis model to obtain a plurality of predicted molecule sets respectively corresponding to a plurality of second leaf nodes, the target retrosynthesis model comprising a graph neural network and a reactant generation network, the graph neural network being configured to predict a bond breaking position of the compound molecule corresponding to the first leaf node, and the reactant generation network being configured to determine a predicted molecule set based on the bond breaking position;
   recursively processing the plurality of predicted molecule sets corresponding to the plurality of second leaf nodes and determining a terminal node that satisfies a preset condition; and
   traversing paths corresponding to the terminal node to determine a retrosynthetic path of the target molecule.

2. The method according to claim 1, wherein the processing the compound molecule corresponding to the first leaf node by using a target retrosynthesis model to obtain a plurality of predicted molecule sets respectively corresponding to a plurality of second leaf nodes comprises:

   determining a first string for the compound molecule corresponding to the first leaf node;
   converting the first string into a first molecular map;
   determining the bond breaking position of the compound molecule corresponding to the first leaf node by processing the first molecular map by using the graph neural network;
   determining at least one synthon according to the bond breaking position by using the reactant generation network; and
   screening the at least one synthon based on a preset rule to determine the predicted molecule set.

3. The method according to claim 2, wherein the determining the bond breaking position of the compound molecule corresponding to the first leaf node by processing the first molecular map by using the graph neural network comprises:

   determining bond breaking information by processing the first molecular map and under a limitation on a number of broken bonds by using the graph neural network; determining a target key feature of the target molecule, the target key feature comprising an atom key feature and a bond key feature, the atom key feature comprising at least one of an atom type, a number of bonds, a formal charge, chirality, a number of hydrogen atoms, an atomic hybridization state, aromaticity, an atomic weight, a high frequency reaction center feature, and a reaction type, and the bond key feature comprising at least one of a bond type, a conjugate, a ring bond, and a molecular stereochemical feature; and
   extracting the key breaking information based on the target key feature to determine the bond breaking position.

4. The method according to claim 3, wherein the graph neural network is trained by:

   obtaining a first training molecule and a first training synthon corresponding to the first training molecule;
   determining a node feature and an edge feature of the first training molecule and the first training synthon, the node feature indicating a relationship of atoms between of the first training molecule and the first training synthon, and the edge feature indicating a relationship of chemical bonds between the first training molecule and the first training synthon;
   determining a first loss function based on the node feature and the edge feature;

determining a bond breaking probability of the chemical bond between the first training molecule and the first training synthon;

determining a second loss function based on the bond breaking probability; and

updating a model parameter of the graph neural network according to the first loss function and the second loss function.

5. The method according to claim 2, wherein the determining at least one synthon according to the bond breaking position by using the reactant generation network comprises:

splitting the target molecule at the bond breaking position to obtain at least one synthon molecular map;

converting the synthon molecular map into a second string;

updating the second string based on a preset reaction type to obtain a third string; and

determining the at least one synthon by processing the third string by using the reactant generation network.

6. The method according to claim 5, wherein the reactant generation network is trained by:

obtaining a second training molecule, a second training synthon, and a training reactant;

determining a first training string based on a string corresponding to the second training molecule, a string corresponding to the second training synthon, and the preset reaction type; determining a second training string corresponding to the training reactant;

associating the first training string and the second training string to determine a first training sample pair; and

training the reactant generation network based on the first training sample pair.

7. The method according to claim 6, further comprising:

obtaining a candidate string predicted by the graph neural network;

adding the candidate string into the string corresponding to the second training synthon to update the first training string to a third training string;

associating the third training string and the second training string to determine a second training sample pair; and

training the reactant generation network based on the second training sample pair.

8. The method according to claim 6, further comprising:

determining a target character format; and

updating the first training string based on the target character format.

9. The method according to claim 1, wherein the recursively processing the plurality of predicted molecule sets corresponding to the plurality of second leaf nodes and determining a terminal node that satisfies a preset condition comprises: for a predicted molecule set among the plurality of predicted molecule sets that corresponds to one of the plurality of second leaf nodes,

determining a first candidate molecule that corresponds to a preset reaction type and that is in the predicted molecule set;

processing the first candidate molecule by using the target retrosynthesis model to obtain a plurality of predicted molecule sets corresponding to a plurality of third leaf nodes;

recursively processing the plurality of predicted molecule sets corresponding to the plurality of third leaf nodes to determine a second candidate molecule; and

determining that a leaf node corresponding to the second candidate molecule is the terminal node in response to the second candidate molecule satisfying the preset condition.

10. The method according to claim 9, wherein the determining that a leaf node corresponding to the second candidate molecule is the terminal node in response to the second candidate molecule satisfying the preset condition comprises:

traversing based on the second candidate molecule to obtain a plurality of pathway molecules; and

determining that the second candidate molecule satisfies the preset condition and determining that the leaf node corresponding to the second candidate molecule is the terminal node, in response to the pathway molecule being a molecule in a basic molecule set.

11. The method according to claim 9, wherein the determining that a leaf node corresponding to the second candidate molecule is the terminal node in response to the second candidate molecule satisfying the preset condition comprises:

determining a number of times of recursive processing corresponding to the second candidate molecule in the tree structure; and

determining that the second candidate molecule satisfies the preset condition and determining that the leaf node corresponding to the second candidate molecule is the terminal node, in response to the number of times of recursive processing reaching a preset value.

12. The method according to claim 1, wherein the target molecule is a drug molecule, the tree structure is a Monte Carlo tree, and a compound molecule corresponding to the second leaf node is obtained by screening based on at least one of functional group interference, use of a protective group, a reaction feature of a functional group, and a name reaction rule.

13. An apparatus for predicting retrosynthesis of a compound molecule, comprising:

an obtaining unit, configured to obtain a target molecule and determining the target molecule as a root node in a tree structure; the root node being associated with a first leaf node in the tree structure, the first leaf node corresponding to a compound molecule, and the tree structure comprising a retrosynthetic path of the target molecule corresponding to the root node;

an expansion unit, configured to processing the compound molecule corresponding to the first leaf node by using a target retrosynthesis model to obtain a plurality of predicted molecule sets respectively corresponding to a plurality of second leaf nodes, the target retrosynthesis model comprising a graph neural network and a reactant generation network, the graph neural network being configured to predict a bond breaking position of the compound molecule corresponding to the first leaf node, and the reactant generation network being configured to determine a predicted molecule set based on the bond breaking position;

a processing unit, configured to recursively process the plurality of predicted molecule sets corresponding to the plurality of second leaf nodes and determine a terminal node that satisfies a preset condition; and

a prediction unit, configured to traverse paths corresponding to the terminal node to determine a retrosynthetic path of the target molecule.

14. A computer device, comprising a processor and a memory,

the memory being configured to store program code; and the processor being configured to execute the method for predicting retrosynthesis of a compound molecule according to any one of claims 1 to 12 based on instructions in the program code.

15. A computer-readable storage medium, storing instructions, the instructions, when run on a computer, causing the computer to execute the method for predicting retrosynthesis of a compound molecule according to any one of claims 1 to 12.

16. A computer program product, comprising instructions, the instructions, when run on a computer, causing the computer to execute the method for predicting retrosynthesis of a compound molecule according to any one of claims 1 to 12.

**FIG. 1**

**FIG. 2**

301

Obtain a target molecule and determining the target molecule as a
root node in a tree structure

302

Process a compound molecule corresponding to a first leaf node
associated with the root node by using a target retrosynthesis
model to obtain multiple predicted molecule sets corresponding to
multiple second leaf nodes

303

Recursively process the multiple predicted molecule sets
corresponding to the multiple second leaf nodes and determine a
terminal node that satisfies a preset condition

304

Traverse paths corresponding to the terminal node to determine a
retrosynthetic path of the target molecule

FIG. 3

**FIG. 4**

**FIG. 5**

**FIG. 6**

**FIG. 7**

Atom
feature
(Node)

h(l)

↓

```
┌─────────────────────┐
│    Linear layer     │
└─────────────────────┘
```

↓

```
┌─────────────────────┐
│   Attention layer   │
└─────────────────────┘
```

↓

h(l+1)

Graph attention
network (GAT)

---

Atom
feature
(Node)

h(l)

Bond
feature
(Edge)

p(l)

↓

```
┌─────────────────────┐
│    Linear layer     │
└─────────────────────┘
```

↓

```
┌─────────────────────┐
│   Attention layer   │
└─────────────────────┘
```

↓

h(l+1)

```
┌─────────────────────┐
│    Linear layer     │
└─────────────────────┘
```

↓

p(l+1)

Edge graph attention
network (EGAT)

## FIG. 8

| Sample string | | Ta |
|---|---|---|
| <RXN_k> Product <LINK> Synthon1.Synthon2 | ⟹ | Reactent1.Reactent2 |
| <RXN_k> Product <LINK> Synthon2.Synthon1 | ⟹ | Reactent2.Reactent1 |

Examples:

| | | |
|---|---|---|
| <RXN_k> BrCCSc1cccs1 <LINK> [CH2]CBr.[S]c1cccs1 | ⟹ | BrCCBr.Sc1cccs1 |
| <RXN_k> BrCCSc1cccs1 <LINK> [S]c1cccs1.[CH2]CBr | ⟹ | Sc1cccs1.BrCCBr |

## FIG. 9

1001

Obtain a target molecule and determining the target molecule as a root node in a tree structure

1002

Expand the first leaf node By using a target retrosynthesis model to obtain multiple second leaf nodes

1003

Obtain a reaction screening requirement in response to a target operation

1004

Screen the compound molecules corresponding to the second leaf node based on the reaction screening requirement

1005

Recursively process the predicted molecule set corresponding to the obtained second leaf nodes and determine a terminal node that satisfies a preset condition

1006

Traverse paths corresponding to the terminal node to determine a retrosynthetic path of the target molecule

FIG. 10

**FIG. 11**

**FIG. 12**

**FIG. 13**

**FIG. 14**

1500

Prediction apparatus

1501

Obtaining unit

1502

Expansion unit

1504

Prediction unit

1503

Pocessing unit

**FIG. 15**

1610 RF circuit

1670 WiFi module

1690 Power supply

1680

1620 Memory

Processor

1660 Audio circuit

Speaker 1661

Microphone 1662

Sensor 1650

Input unit

1631

Touch panel

1630

Other input device

1632

Display unit

1640

Display panel

1641

**FIG. 16**

FIG. 17

## INTERNATIONAL SEARCH REPORT

| | International application No. |
|---|---|
| | **PCT/CN2022/073158** |

| **A.** | **CLASSIFICATION OF SUBJECT MATTER** |
|---|---|

G16C 20/10(2019.01)i

According to International Patent Classification (IPC) or to both national classification and IPC

| **B.** | **FIELDS SEARCHED** |
|---|---|

Minimum documentation searched (classification system followed by classification symbols)

G16C,G06N,H04L

Documentation searched other than minimum documentation to the extent that such documents are included in the fields searched

Electronic data base consulted during the international search (name of data base and, where practicable, search terms used)

WPI, EPODOC, CNPAT, CNKI, IEEE: 化合物, 反应物, 分子, 逆合成, 反合成, 人工智能, 机器学习, 树, 蒙特卡洛树, 根节点, 路径, 路线, 叶节点, 叶子节点, 遍历, 图神经网络, 子图, 断键, 断裂, 断开, 递归, 选择, 扩展, 模拟, 更新, compounds, reactant, molecule, retrosynthesis, artificial intelligence, machine learning, tree, MCT, root, path, leaf, node, traverse, graph neural network, GNN, subgraph, break, recursion, select, extension, simulation, update

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | 郭世豪 (GUO, Shihao). "基于深度学习的化合物逆合成系统设计与实现 (Design and Implementation of Compound Retro Synthesis System Based on Deep Learning)" 中国优秀博硕士学位论文全文数据库(硕士)工程科技I辑 (Science-Engineering (A), Chinese Selected Doctoral Dissertations and Master's Theses Full-Text Databases (Master)), No. 08, 15 August 2020 (2020-08-15), ISSN: 1674-0246, abstract, chapters 3-4 | 1-16 |
| A | CN 111524557 A (TENCENT TECHNOLOGY SHENZHEN CO., LTD.) 11 August 2020 (2020-08-11) entire document | 1-16 |
| A | CN 112037868 A (TENCENT TECHNOLOGY SHENZHEN CO., LTD.) 04 December 2020 (2020-12-04) entire document | 1-16 |
| A | CN 111223532 A (TENCENT TECHNOLOGY SHENZHEN CO., LTD. et al.) 02 June 2020 (2020-06-02) entire document | 1-16 |

☑ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 April 2022** | **19 April 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/073158**

### C. DOCUMENTS CONSIDERED TO BE RELEVANT

| Category* | Citation of document, with indication, where appropriate, of the relevant passages | Relevant to claim No. |
|---|---|---|
| A | US 2009232031 A1 (VASSEUR, Jean-Philippe et al.) 17 September 2009 (2009-09-17)<br>    entire document | 1-16 |
| A | 刘伊迪 等 (LIU, Yidi et al.). "机器学习在有机化学中的应用 (Application of Machine Learning in Organic Chemistry )"<br>有机化学 (*Chinese Journal of Organic Chemistry*),<br>Vol. 40, No. 11, 30 November 2020 (2020-11-30),<br>ISSN: 0253-2786,<br>    entire document | 1-16 |

Form PCT/ISA/210 (second sheet) (January 2015)

**INTERNATIONAL SEARCH REPORT**
Information on patent family members

International application No.

**PCT/CN2022/073158**

| Patent document cited in search report | | | Publication date (day/month/year) | Patent family member(s) | Publication date (day/month/year) |
|---|---|---|---|---|---|
| CN | 111524557 | A | 11 August 2020 | None | |
| CN | 112037868 | A | 04 December 2020 | None | |
| CN | 111223532 | A | 02 June 2020 | None | |
| US | 2009232031 | A1 | 17 September 2009 | None | |

Form PCT/ISA/210 (patent family annex) (January 2015)

**REFERENCES CITED IN THE DESCRIPTION**

**Patent documents cited in the description**

- CN 2021101122078